(19) Europäisches Patentamt
European Patent Office
Office européen des brevets

(11) **EP 4 166 576 A1**

(12) **EUROPEAN PATENT APPLICATION**

(43) Date of publication:
**19.04.2023 Bulletin 2023/16**

(21) Application number: **21202228.9**

(22) Date of filing: **12.10.2021**

(51) International Patent Classification (IPC):
**C08B 15/02** (2006.01)    **C08L 1/04** (2006.01)

(52) Cooperative Patent Classification (CPC):
**C08B 15/02; A21D 2/188; A23L 29/262;
A23L 33/24; A61K 31/717; A61P 1/00; C08L 1/04**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Katholieke Universiteit Leuven
3000 Leuven (BE)**

(72) Inventor: **The designation of the inventor has not
yet been filed**

(54)    **SHORT AMORPHOUS CELLULOSE POLYMERS**

(57)    The invention relates to methods of producing a microcrystalline cellulose, the method comprising the steps of:
a) providing a composition comprising at least 60 % (w/w) cellulose, wherein the cellulose has an average Degree of Polymerization (DP) of between 20 and 100,
b) mechanically treating said composition until the crystallinity index of said cellulose is below 0.5 and the degree of polymerization is below 100.

Figure 6

EP 4 166 576 A1

**Description**

**FIELD OF THE INVENTION**

The invention relates to the processing of cellulose material and its use as dietary fiber.

**BACKGROUND OF THE INVENTION**

Cellulose, a β-1,4-bond glucose polymer, is the most abundant renewable material in nature, being the main building block of the plant cell wall. Here, linear glucose polymers are closely packed with strong hydrogen interactions between the different polymers, resulting in long rigid (crystalline) cellulose fibers [Chen (2005), Biotechnology of Lignocellulose]. Today, cellulose is of growing interest for high-end valorization: they can be used as glucose feedstock in the biorefinery or as bio-based fiber for its distinct mechanical properties [Cherubini (2010) Energy Conversion and Management 51(7), 1412-1421; Bozell and Petersen(2010) Green Chemistry 12(4), 5392]. Refined cellulose fibers, such as microcrystalline cellulose or nanocelluloses, are purified and partially hydrolyzed, and are widely used in, e.g. packaging material, coatings, membranes, analytics, construction material, drug delivery and/or in the food industry. The crystallinity of these fibers (mostly obtained after homogenization) should be high enough to ensure the strength of the fiber compositions [Brinchi et al. (2013) Carbohydrate polymers 94(1), 154-169; An et al. (2016), Cellulose 23(4), 2409-2420], and therefore, at the moment, decreasing the crystallinity of microcrystalline cellulose or nanocellulose is not targeted. However, it is our strong belief that decreasing the crystallinity of the refined fibers can be relevant for unexploited valorizations, where high accessibility of the fibers would be advantageous. The crystallinity of the cellulose fibers (partially hydrolyzed or native) is nevertheless one of the main reasons cellulose is difficult to degrade [Xu et al. (2012) Biotechnology for Biofuels 5(1), 58].

In the human gut, for example, cellulose is not fermented in the large intestine, as colon microbiota cannot access the β-1,4-bonds of the glucose-polymers [Cummings et al. (1984) Gut 25(8), 805-810]. However, fermentation of dietary fibers like cellulose in the colon is correlated with different positive health effects, linked to the production of short-chain fatty acids (SCFA), which provides the colon cells with energy and contribute to a balanced glucose and cholesterol metabolism [den Besten et al. (2013) J. lipid res. 54(9), 2325-2340]. In addition, fermentation of carbohydrates causes a decrease in the pH in the lumen and repression of pathogen growth [Wong et al. (2006) J. Clin. Gastroenterology 40(3), 235-243].

Since the average daily intake of dietary fiber is (still) too low in current Western diets ["Scientific Opinion on Dietary Reference Values for Carbohydrates and Dietary Fibre" (2010) EFSA Journal 8(3), 1462], the food industry is continuously searching for valuable dietary fiber components. Existing fiber additives (e.g. wheat bran) are often linked with organoleptic and/or techno-functional shortcomings [Hemdane et al. (2015) Food Chemistry 187, 280-289], while new introductions (e.g. arabinoxylan-oligosaccharides from wheat bran) have been repressed by too high production costs [Swennen et al. (2006) J. Sci. Food and Agriculture 86(11), 1722-1731]. The search for new fiber additives is, therefore, still very relevant.

The exploitation of cellulose for this purpose will have a large impact due to the abundant character of the material. Cellulose is already used today as a low-fat substituent, bulking agent, thickener and/or emulsion-stabilizer [Mirhosseini et al., (2008) Carbohydrate Polymers 73(1), 83-91], but the physiological health benefits of cellulose are limited to an increased fecal bulk, due to the recalcitrance of the fibers.

**Summary**

The present invention provides a food-grade modification strategy of cellulose towards higher overall accessibility in order to increase the fermentability of cellulose in the colon.

A carbohydrate fiber composition containing accessible cellulose structures is generated by the combination of mechanical impact and chemical hydrolysis with organic acids. A subsequent mechanical post-treatment of these short cellulose fibers after hydrolysis improves the physiological effects of the fiber composition for use in the food industry, as their fermentability in the colon is increased. *In vitro* fermentation experiments with the cellulose obtained after this triple treatment showed that the pH decreased, protein fermentation was inhibited, and the proportion of butyrate production was enhanced, compared to cellulose which underwent a single mechanical treatment and hydrolysis.

The present invention allows decreasing the recalcitrance of cellulose (such as Avicel) in a food grade way, without losing the insoluble character of the fibers. In a particular embodiment, a planetary ball mill treatment, chemical hydrolysis with citric acid, and an additional ball mill treatment resulted in fibres with a crystallinity index below 0.5 and an average DP between 20 AGU and 50 AGU. The recalcitrance of these short amorphous cellulose (SAC) fibers was decreased since these fibers were proven to be more accessible for enzymatic blends than unmodified cellulose (<0.4 conversion factor after 1h hydrolysis with CTec2 enzyme blend).

This enhanced accessibility results in enhanced health benefits when the cellulose is ingested, thanks to an enhanced fermentation in the large intestine. The minimal fermentation degree of 7.6% for prior art cellulose could be enhanced towards at least 45,8%, based on an *in vitro* fermentation experiment.

The invention is further summarized in the following statements:

1. A method of producing a microcrystalline cellulose, the method comprising the steps of:
a) providing a composition comprising at least 60 % (w/w) cellulose, wherein the cellulose has an average Degree of Polymerization (DP) of between 20 and 100, b) mechanically treating said composition until the crystallinity index of said cellulose is below 0.5 and the degree of polymerization is below 100.
2. The method according to statement 1, wherein in step a, said composition comprises at least 70, 80, or 90 % (w/w) cellulose.
3. The method according to statement 1 or 2, wherein in step b) the composition is treated until the crystallinity index of said cellulose is below 0.45, below 0.40 below 0.35 or below 0.33.
4. The method according to any one of statements 1 to 3, wherein in step b) the composition is treated until the average degree of polymerization is below 80, below 60, below 50 below 40 or below 30.
5. The method according to any one of statements 1 to 4, wherein in step b) the composition is treated until the crystallinity index of said cellulose is below 0.33 and the average degree of polymerization is below 50.
6. The method according to any one of statements 1 to 5, wherein the composition in step a) is obtained via a method of mechanically treating a composition comprising cellulose and a subsequent method of hydrolyzing said mechanically treated composition.
7. A composition comprising at least 60 wt % cellulose, wherein the cellulose has a degree of Polymerization (DP) below 100 and a crystallinity index below 0,5.
. The composition according to statement 7, The method according to statement 1, wherein in step b) the composition is treated until the crystallinity index of said cellulose is below 0.45, below 0.40 below 0.35 or below 0.33.
9. The composition according to statement 7 or 8, wherein the average degree of polymerization of said cellulose is below 80, below 60, below 50, below 40 or below 30.
10. The composition according to any one of statements 7 to 9, wherein the crystallinity index of said cellulose is below 0.33 and the average degree of polymerization is below 50.
11. The composition according to any one of statements 7 to 10 comprising at least 70, at least 80 or at least 90 % cellulose.
12. A food product comprising between 1 and 50 wt % of a composition according to any one of statements 7 to 11.
13. The food product according to which is a cereal product.
14. Use of a composition according to any one of statements 7 to 13 as a dietary fiber in a food composition.
15. A method of producing a microcrystalline cellulose, the method comprising the steps of:

a) mechanically treating a composition comprising cellulose,
b) hydrolyzing said mechanically treated composition,
c) further mechanically treating said hydrolyzed composition until the crystallinity index of said cellulose is below 0.5 and the average degree of polymerization is below 100.

16. The method according to statement 15, wherein in step a), said composition comprises at least 70, at least 80, or at least 90 % (w/w) cellulose.
17. The method according to statement 15 or 16, wherein in step c) the composition is treated until the crystallinity index of said cellulose is below 0.45, below 0.40 below 0.35 or below 0.33.
18. The method according to any one of statements 15 to 17, wherein in step c) the composition is treated until the average degree of polymerization is below 80, below 60, below 50 below 40 or below 30.
19. The method according to any one of statements 15 to 18, wherein in step c) the composition is treated until the crystallinity index of said cellulose is below 0.33 and the average degree of polymerization is below 50.

## DETAILED DESCRIPTION

Figure legends

Figure 1: XRD refractogram with the 1002 peak and the Iam minimum to define the Crystallinity index.
Figure 2: Crystallinity index of Avicel cellulose after a mechanical pre-treatment in a planetary ball mill (BM).
Figure 3: Average DP of modified cellulose in function of the insoluble yield. Modifications consist of a ball mill treatment (open squares), an acid hydrolysis under variable process conditions (red dots), or the same acid hydrolysis procedures after a ball mill pre-treatment of 6h at 500 rpm (black dots).

Figure 4: Average DP of ball-milled Avicel cellulose, in function of the treatment time.

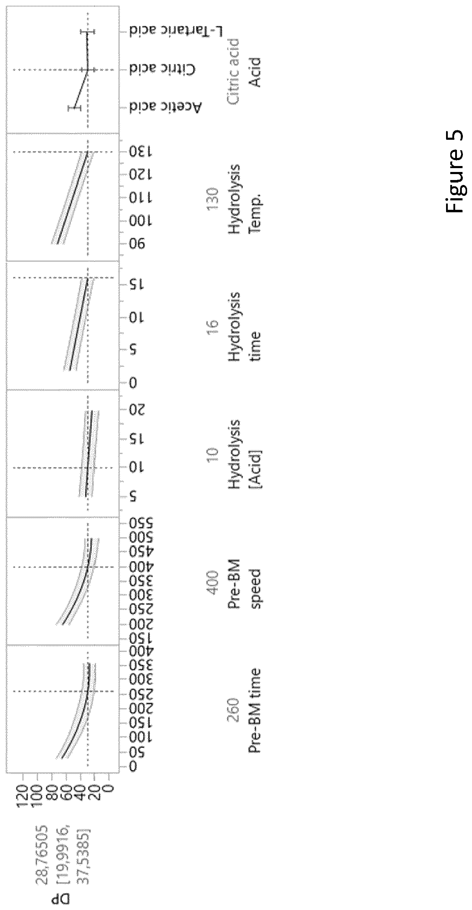

Figure 5: Prediction profiler of the response surface model (r2=0.95), which describes the dependency of average DP on different process parameters. The grey numbers indicate the process parameters of SCC/SAC29.

Figure 6: Average DP for unmodified and modified celluloses.

Figure 7: SEM pictures of Avicel, BM (ball milled for 260min at 400rpm), SCC29 and SAC29.

Figure 8: D50 values for the modified celluloses, measured with a laser diffraction particle size analyzer.

Figure 9: Strong water retention capacity of unmodified and modified celluloses

Figure 10: Cellulose conversion for modified and unmodified Avicel, during a 1h digestion with Cellic CTec2 enzyme blend at 40°C. High enzymatic accessibility of the cellulose results in a high conversion degree.

Figure 11: pH of a fecal slurry with cellulose additions during fermentation at 37°C. SCFA production, induced by cellulose fermentation, decreases the pH.

Figure 12: Production of Acetate, Propionate and Butyrate during the in vitro fermentation.

Figure 13: Production of Acetate, Propionate and Butyrate during the in vitro fermentation.

Figure 14: pH of a fecal slurry with cellulose additions during fermentation at 37°C. SCFA production, induced by cellulose fermentation, decreases the pH.

**Definitions**

*Avicel:* Commercial cellulose preparation, with an average DP of around 160 AGU, and a crystallinity index (CI) of 0.72-0.87 (depending on resource).

*BM:* Cellulose, treated in a ball mill [treatment A]. These ball-milled celluloses have a CI under 0.33, and DP of 50-160 AGU (depending on the severity of the treatment).

*SCC fiber:* Cellulose after a consecutive ball mill and acid hydrolysis treatment [treatment A+B]. These fibers have a DP<100, and CI above 0.50

*SAC fiber:* Cellulose after a consecutive ball mill, acid hydrolysis and another ball mill treatment [treatment A+B+C]. These fibers have a DP<100, and CI under 0.33

**Materials and methods**

**Process description/optimization**

Avicel PH-101 (Sigma-Aldrich, Deurne, Belgium) [DP about 160 AGU, and a crystallinity index (CI) between 0.72 and 0.87] was modified towards short crystalline cellulose fibers (SCC fibers), by a subsequent combination of a treatment in the planetary ball mill pre-treatment (PM100, Retch GmbH) and an acid hydrolysis with mild organic acids (Sigma-Aldrich, Deurne, Belgium). After washing and drying, the SCC fibers were treated in the ball mill another time to produce short amorphous cellulose (SAC) fibers, which were expected to be more accessible for enzymatical or chemical reactions. To optimize and understand the modification process, different process parameters were varied, and the end products were evaluated on their average degree of polymerization (DP), crystallinity, and process yield. Afterwards, a selection of 10 samples was made for further analysis and valorization examples.

Both **ball mill** treatments (batches of 20g) were conducted in a ball mill sample holder with a Zirconium oxide coating. Milling conditions prior to the hydrolysis procedure (referred to as "ball mill pre-treatment") were varied in time and speed between 30m and 6h, and 200rpm-500rpm, respectively. On the contrary, the ball mill treatment after hydrolysis ("ball mill post-treatment") was always performed at 500rpm (0.5h-1h).

**Hydrolysis** was performed in an acid in water solution, with a solid to liquid ratio of 4.8%. Therefore, mild organic acids (hydrochloric acid (HCl), Oxalic acid (OA), Tartaric acid (TA), Citric acid (CA) and Acetic acid (AA)) were used in concentrations that varied between 2.5% and 50%, and hydrochloric acid was used in a concentration of 0.0005%. The temperature during these hydrolyses was varied between 90°C and 130°C, while the stirring rate was always fixed at 800 rpm.

After hydrolysis, the insoluble material was washed with water until neutral pH, and dried in an oven for 48h at 60°C, before undergoing the ball mill post-treatment. Alternatively, the SCC fiber suspension was freeze-dried, after a snap freezing step with liquid nitrogen, or spray-dried before being treated in the ball mill.

For the first screening experiments, hydrolysis was conducted in cellulose batches of 0.3g, while the optimization of the production process parameters was performed with 15g batches. For the process optimization, an I-optimal full factorial experimental design with response surface methodology was employed, using the JMP package. The six-factorial-three-level design with Ball mill speed, Ball mill time, Hydrolysis temperature, Hydrolysis time, Acid concentration and type of acid as input parameters required 42 experiments.

After optimization, a sample set of 10 celluloses (unmodified Avicel, ball-milled Avicel, 4 SCC fibers and 4 SAC fibers) was selected for further characterization and evaluation of the functionalities (examples). The Ball mill pre-treatment was performed for 260 minutes at 400rpm, while the hydrolysis was always performed with a 10% citric acid solution, with varying hydrolysis time and temperature (Table 1). The distinction between the SAC and SCC fibers consists of the absence or presence of a BM post-treatment (30m at 500rpm) or not.

**Table 1: Hydrolysis conditions of the selected SCC and SAC fibers**

|  | Hydrolysis time (h) | Hydrolysis Temperature (°C) |
|---|---|---|
| SCC29/SAC29 | 16 | 130 |
| SCC50/SAC50 | 16 | 110 |
| SCC75/SAC75 | 2 | 110 |
| SCC97/SAC97 | 2 | 90 |

**Characterization**

De **average degree of polymerization** ($DP_v$) of the cellulose is defined as the average length (expressed in anhydroglucose units (AGU)) of the glucose polymers in a cellulose fiber. This $DP_v$ is determined viscometrically with a capillary viscometer, based on an NF G 06-037 norm of the French institute for normalization (AFNOR). Purified cellulose samples (0,075g) are dissolved in a 0,5M Bis(ethylenediamine)cupper(II)hydroxide solution (15ml), and the viscosity of this solution at 25°C is measured with a Schott Geräte capillary viscometer, type nr. 509 04. The $DP_v$, expressed in anhydroglucose units [AGU], is calculated from the boundary viscosity of the solution ($\eta$), based on an empirical relation: $DP_v^\alpha = \frac{\eta}{K'}$, where $\alpha$ and K are empirical constants, equal to 1 and $7{,}5.10^{-3}$ respectively. The boundary viscosity $\eta$ is determined from $\eta_a = \eta.C.10^{(0,14.\eta.C)}$, where $\eta_a$ is the specific viscosity of the solution, and C is the concentration of cellulose per 100 ml.

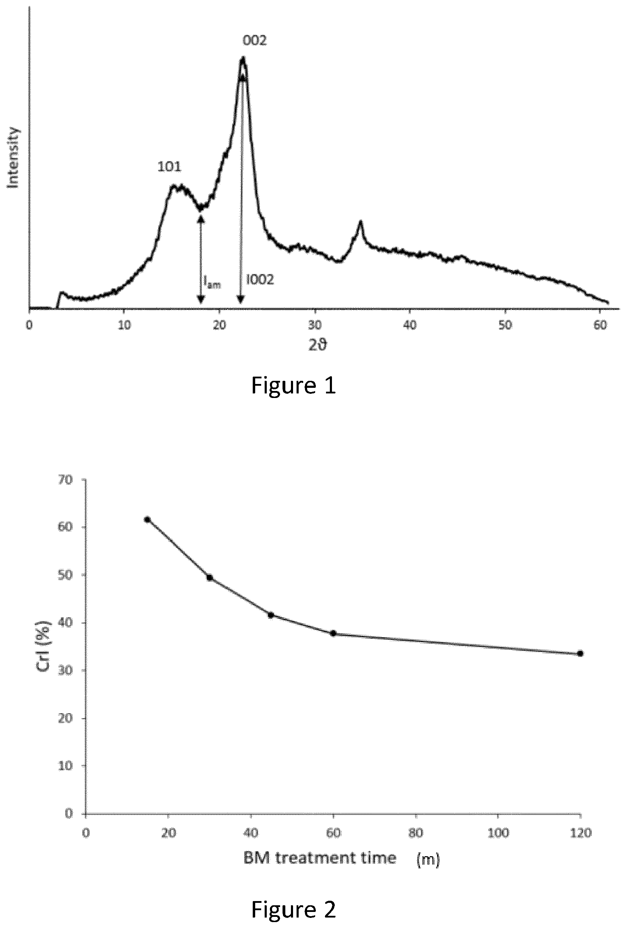

The **crystallinity index (CI)** is determined with x-ray diffraction (XRD) measurements based on the peak height method developed by Sega et al. (1959) Textile Res. J. 29(10), 786-94]. The CI is calculated from the ratio of the height of the 002 peak ($I_{002}$) and the height of the minimum between the $I_{002}$ and the 101 peaks ($I_{am}$) around 18°, as shown in Figure 1. When a crystallinity index of 0.33 is reached, the $I_{am}$-minimum is not visible anymore. Amorphous samples of which the $I_{am}$-minimum is not possible anymore, are getting a CI of <0.33.

The **aggregate size** and **aggregate shape** influence the accessibility of the cellulose since the reactive surface largely depends on these characteristics [Gusakov (2007) Biotechnology and Bioengineering 97(5), 1028-1103]. The aggregate size of the cellulose is determined with an LS 13320 laser diffraction particle size analyzer (Beckman Coulter). A He-Ne laser beam is emitted on a solution of the cellulose in water, and the volumetric PSDs are calculated from the intensity profile of the scattered light with the Mie theory using the instrument's software. Furthermore, the agglomerates are visualized with a scanning electron microscope (SEM). A JEOL JSM-6010 JV microscope is used after coating the cellulose with a JEOL JSC-1300 sputter. The **enzymatic digestibility** is determined by calculating the enzymatic conversion (EC) after reaction with Cellic CTec2 cellulase, as described by Chen et al. (2015) Applied Energy 150, 224-232]. Cellulose is suspended (1 %w/v) in a 50mM Sodium acetate buffer (pH 4.8), and stirred at 900 rpm together with 20U/g cellulose Cellic CTec2 cellulase (Sigma-Aldrich, Deurne, Belgium). After 1 hour of incubation at 40°C, the enzymes are denatured by heating the solution (5min@110°C) and the solid fraction is separated from the supernatant by centrifuging at 5000G. The amount of glucose and cellobiose in the supernatant, which is formed from cellulose hydrolysis, is determined by High-performance-anion-exchange chromatography with pulsed amperometric detection (HPAEC-PAD) on a Dionex ICS3000 chromatography system (Sunnyvale, CA, USA). Saccharides were separated on a CarboPac PA-100 column (4x250 mm), equilibrated with 90 mM NaOH, by applying a sodium acetate gradient. The enzymatic conversion (%) is calculated from the amount of glucose ($m_g$) and cellobiose ($m_{cb}$) in the supernatant, and the amount of starting substrate ($m_c$):

$$EC\ (\%) = \frac{m_g + m_{cb}}{1.1 m_c}$$

**In *vitro* fermentation tests** were performed, following the procedure of De Preter et al (2010) Molecular nutrition & food research 54(12), 1791-1801. In this set-up, 100mg of the cellulose fibers are added to 25 ml of a 10 wt% fecal slurry, consisting of human feces from 8 donors in phosphate-buffered saline (pH 7.3). The tubes are incubated anaerobically at 37°C for 48h, under continuous shaking. During this incubation, the colon microbiota from the feces ferment the (accessible) carbohydrate fibers under ideal circumstances, resulting in the production of short-chain fatty acids. After incubation, the pH of the slurry is measured with a Hanna Instruments HI 9025 digital pH meter, and the production of short-chain fatty acids (SCFA) is quantified.

Only the amounts of acetate, propionate, butyrate, isobutyrate and isovaleric acid in the fecal slurry are determined with the gas-chromatographic method of Van de Wiele et al. (2007) J. Applied Microbiology 102(2), 452-460. In this procedure, salts of the SCFA are extracted in diethyl ether. To create and neutralize the salts of the fatty acids, a 25% (w/v) NaOH and 50% sulfuric acid solution is added to the fecal slurry before extracting the SCFA from the fecal slurry to the diethyl ether phase. An Agilent 6890 Series gas chromatograph is used with an EC-1000 Econo-Cap column (25 m × 0,53 mm, 130°C, 1,2 $\mu$m film thickness) with helium (20 ml/min) as carrier gas. A flame ionization detector at 195°C measured the different fatty acids.

## Results

### Process optimization for SCC fibers

A decrease in the average DP is obtained by acid hydrolysis. Acid hydrolysis preferably takes place in amorphous zones of cellulose, which can be incorporated randomly in the crystalline chains of Avicel cellulose by a ball mill pre-treatment [Zhao et al. (2012) Biofuels, Bioproducts and Biorefining 6(4), 465-482; Lin et al. (2010) Applied biochemistry and biotechnology 162(7), 1872-1880]. Figure 2 shows the decrease in crystallinity index (CI) during a ball mill treatment at 500rpm, in function of the treatment time. After a treatment of 120 minutes, the crystallinity index was 0.33, which is at the lower limit of the peak height method of Segal *et al.* If more amorphicity is induced in the fibers, the amorphous signal in the X-ray diffraction is overlaying the crystalline peaks, making the $I_{am}$ valley invisible.

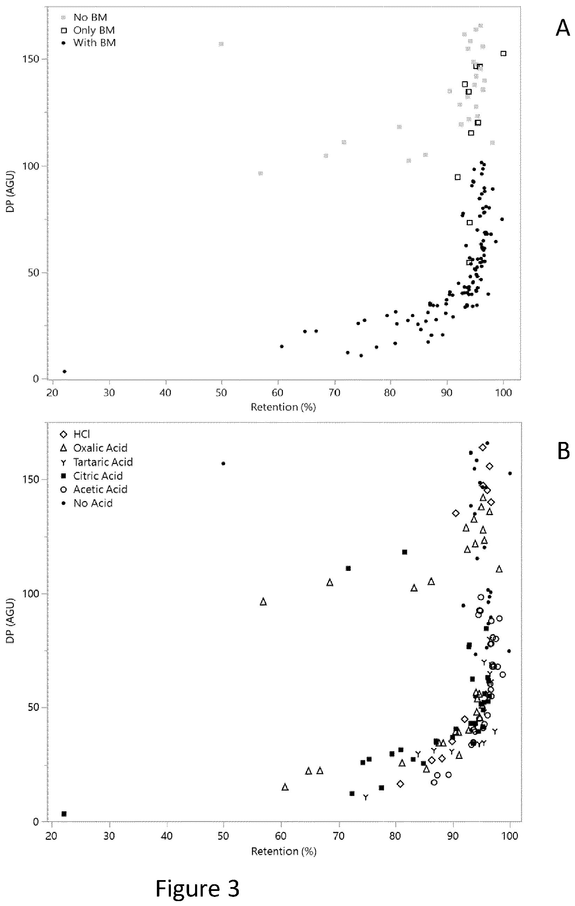

The effect of this decrease in crystallinity, prior to the acid hydrolysis is shown in Figure 3. Herein, untreated Avicel (Average DP of 160 anhydroglucose units (AGU)) and ball milled Avicel (6h at 500rpm) was hydrolyzed with HCl, oxalic acid, tartaric acid, citric acid or acetic acid under different processing conditions (acid concentrations 0.0005%-50%, hydrolysis temperatures 100°C-120°C, hydrolysis time 0.5-16h). The average DP of the insoluble fibers after modification is given in function of their insoluble yield. This insoluble yield is important, since part of the cellulose chains are lost through solubilization of the shortest fibers (DP 1-8) [Shrotri et al. (2013) Green Chemistry 15(10), 2761-2768]. The open squares represent the cellulose fibers after just a ball mill treatment (0.25h-48h, 500 rpm), while the grey squares represent cellulose fibers after a hydrolysis procedure, without the prior ball mill pre-treatment.

For any type of acid, ball mill pre-treatment is necessary to obtain a high yield of SCC fibers with a DP, lower than 90 AGU. Inducing amorphous zones in the long fibers during the pre-treatment results in a lower Level-off degree of polymerization (LODP), which is the lowest average DP that can be reached, without a significant loss in insoluble material. This average DP is typically reached when the easily accessible (amorphous) zones are hydrolyzed, and the acid starts to hydrolyze crystalline zones very slowly [Calvini (2005) Cellulose 12, 445-472].

A hydrolysis procedure without the ball mill pre-treatment is, hence, not suitable for the production of SCC fibers with a DP<50 AGU with yields above 90%, neither is a ball mill treatment on its own.

**Table 2: Effect summary of the response surface model (r²=0.95), which describes the dependency of average DP on different process parameters**

| Input parameter | LogWorth | PValue |
|---|---|---|
| Hydrolysis Temp. | 13,826 | 0,00000 |
| Pre-BM time | 13,048 | 0,00000 |
| Pre-BM speed | 11,990 | 0,00000 |
| Hydrolysis time | 8,169 | 0,00000 |
| Acid | 5,752 | 0,00000 |
| Pre-BM time*Pre-BM time | 2,722 | 0,00190 |
| Pre-BM speed*Pre-BM speed | 2,364 | 0,00432 |
| Hydrolysis [Acid] | 2,056 | 0,00878 |

Table 2 shows that the temperature during hydrolysis is of importance for decreasing the average DP of the insoluble fibers. Also the presence and intensity of the ball mill pre-treatment are of importance to ensure efficient hydrolysis towards low DP values.

The weaker monocarboxylic acid, acetic acid (pKa = 4.75), is less efficient in decreasing the average DP of the cellulose in comparison to the dicarboxylic and tricarboxylic acids (L-Tartaric acid and Citric acid). A stronger acid such as citric acid or tartaric acid is typically used to shorten the processing time but does not influence the insoluble yield.

**Characterization of Avicel, BM, SCC and SAC fibers**

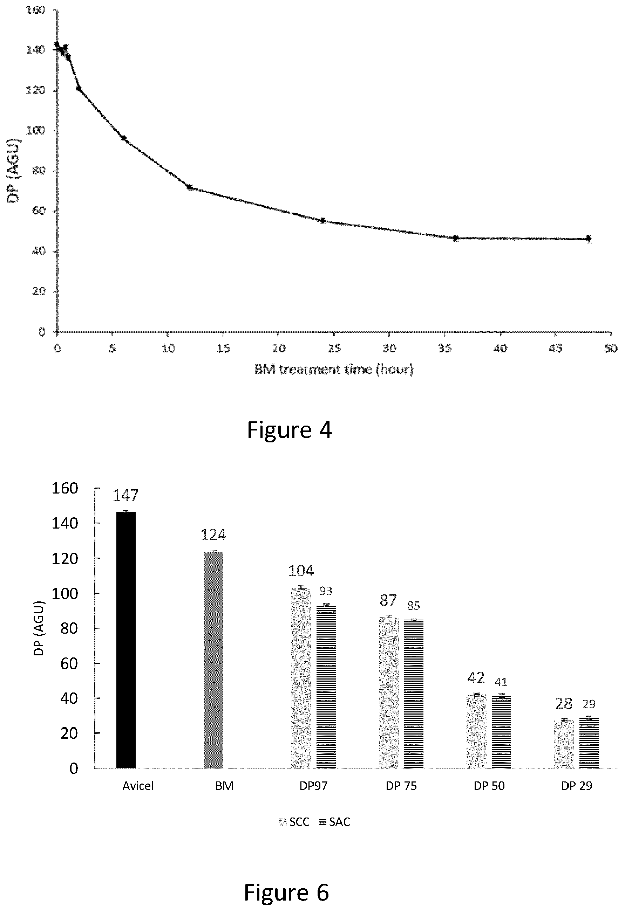

The starting material Avicel has a measured average degree of polymerization of 140-170 AGU [Deneyer et al. (2016) Green Chemistry 18(20), 5594-5606]. This average DP is lowered through the modification in the first ball mill treatment towards 124AGU (Figure 6). The combination of a BM pre-treatment and an acid hydrolysis with citric acid allows decreasing the average DP further towards 29AGU with an insoluble yield of >98%.

The further ball mill post-treatment has a limited effect on the average DP of the insoluble fibers obtained after hydrolysis. However, the ball mill post-treatment further decreases the crystallinity of the cellulose fibers, as shown in table 3.

A ball mill post-treatment of only 30min allows to break down the crystallinity of SCC fibers, since the XRD analysis only could detect amorphous signal, resulting in a crystallinity index <0.33.

**Table 2: Crystallinity index for Modified celluloses**

|  | Crystallinity index |
| --- | --- |
| Avicel | 0.72 |
| BM | <0.33 |
| SCC96 | 0.51 |
| SCC75 | 0.47 |
| SCC50 | 0.56 |
| SCC29 | 0.62 |
| SAC29-SAC50-SAC75-SAC96 | <0.33 |

**Table 4: Overview of the variations on SCC and SAC, with their corresponding treatment.**

|  | Avicel | BM | SCC | SAC |
| --- | --- | --- | --- | --- |
| DP [AGU] CI [] | ±160  0.70-0.90 | 124  <0.33 | 29,50,75 or 97 (SCC29,SCC50,SCC75, SCC97 respectively)  0.50-0.80 | 29,50,75 or 97 (SAC29,SAC50,SAC75, SAC97 respectively)  <0.33 |
| Treatment | None | Ball mill 260min @ 400rpm | Ball mill and Acid hydrolysis (10% Citric acid) SCC29: 16h, 130°C SCC50: 16h, 110°C SCC75: 2h, 110°C SCC97:2h, 90°C | Ball mill, Acid hydrolysis and subsequent Ball mill subsequent Ball mill: 30min @500rpm |

**Aggregate structure**

SAC fibers differ from Avicel, BM and SCC fibers due to a combination of both low average DP and low crystallinity. These fibers, however, are organized in larger aggregates, and the modification is also influencing the shape, size and properties of these aggregates.

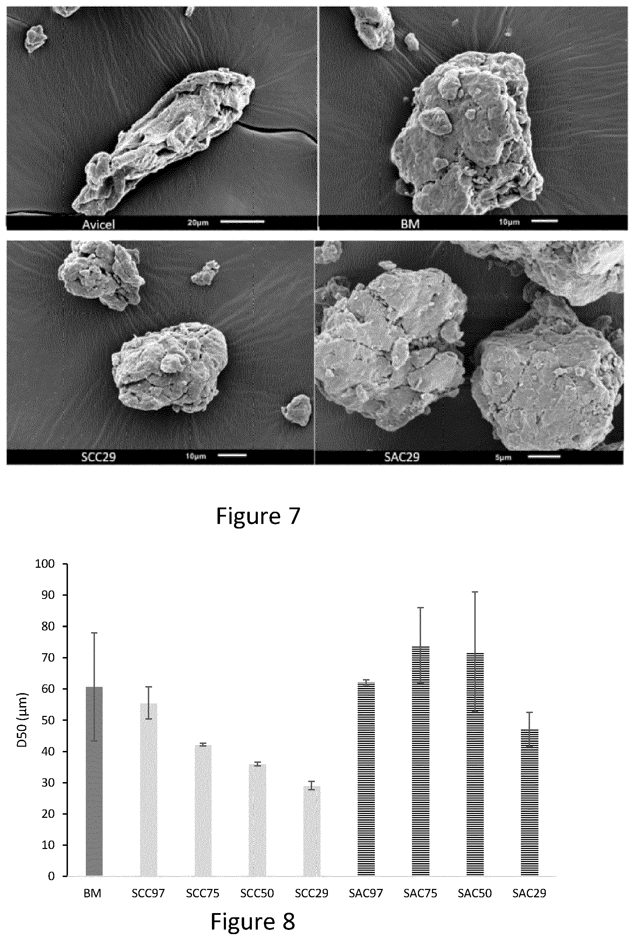

The aggregate shape of unmodified Avicel, ball-milled Avicel, SCC29 and SAC29 are shown in figure 7. For the starting Avicel material, the cellulose fibers are organized in rod-like aggregates with an irregular surface. Dense fiber bundles are still visible on the surface of the aggregates, as well as large macropores between them. A ball mill treatment converts these rod-like aggregate shapes into spherical aggregates. Macropores at the surface disappear through the systematic disruption and reaggregation of the cellulose fibers, and this disappearance already showed up after 15 minutes in the ball mill. However, further treatment time still causes further reorganization of the fibers within the spherical aggregates. The density of the fibers seems higher since the surface is denser, and no macropores are visible anymore. The average particle size is decreased by ball milling, through spheres with an average diameter of 60,7$\mu$m (figure 8). Hydrolysis of the ball-milled cellulose had limited influence on the aggregate shape, neither on the appearance of the aggregate surface. However, the hydrolysis of the cellulose caused a further decrease in particle size, depending on the severity of the hydrolysis (figure 8).

The conversion of the SCC to the SAC is linked to an increase in particle size again, thanks to the compressions occurring in the ball mill. The average diameter is increased towards 47.1pm for the SAC29, and this effect is even more pronounced for the longer SAC's. The shape of the aggregates is not influenced through this additional ball mill step: the dense spherical aggregates with regular surface stay visible on the SEM pictures.

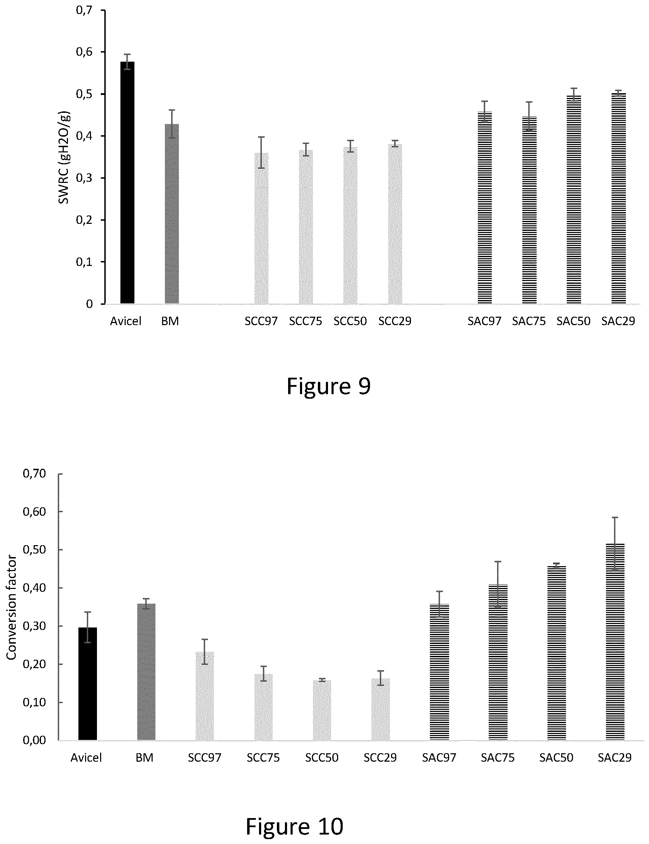

The changes in aggregate structure are translated into a different behavior of water, when it is in contact with the celluloses. In figure 9, the strong water retention capacity(SWRC) of the celluloses is depicted. Unmodified Avicel has the highest SWRC (0.577 g $H_2O$/g $\pm$ 0.018) due to the presence of macropores wherein water is entrapped easily. Disruption of those microfibers and macropores causes a decrease in SWRC for the ball-milled sample. Hydrolysis of the ball-milled cellulose is causing a further decrease, which feeds the suggestion that also a measurable amount of water is bound within the amorphous regions of the cellulose, which disappear during the hydrolysis. Furthermore, the amorphous zones are returning in the conversion from SCC to SAC, which increases water binding again.

**Functionalities**

***Digestibility/fermentability***

Due to the unique combination of the lower DP and crystallinity, enhanced overall accessibility can be expected for the SAC fibers, in comparison to prior art microcrystalline cellulose [Liao et al. (2020) Molecular Catalysis 487, 110883]. In figure 10, the cellulose conversion after a 1h digestibility experiment with a CTec2 cellulase blend is shown. High accessibility for the cellulases will result in a high conversion factor, while the most recalcitrant fibers will have the lowest conversion factor. For unmodified Avicel cellulose, 30,0% $\pm$ 4.0 of the cellulose was converted to glucose and cellobiose after 1h digestion, and a ball mill treatment was already increasing this conversion factor. The SCC fibers are all less digested than unmodified or ball-milled Avicel, showing that a decrease in average DP is not sufficient for improving the enzymatical accessibility of cellulose. The compaction during the ball mill and the removal of amorphous zones during the acid hydrolysis apparently have more effect on the digestibility than the DP decrease, which is expected to influence the digestibility positively. However, if amorphous zones are incorporated in the SCC fibers again (SAC fibers) through the ball mill post-treatment, it is clear that the enzymatic accessibility is exceeding the values of unmodified Avicel or BM cellulose.

Depending on the average DP, the digestibility with the CTec2 enzyme blend can be increased to 52.6% $\pm$ 6.9. Therefore, the combination of both low DP and low crystallinity allows enhancing the accessibility of the cellulose.

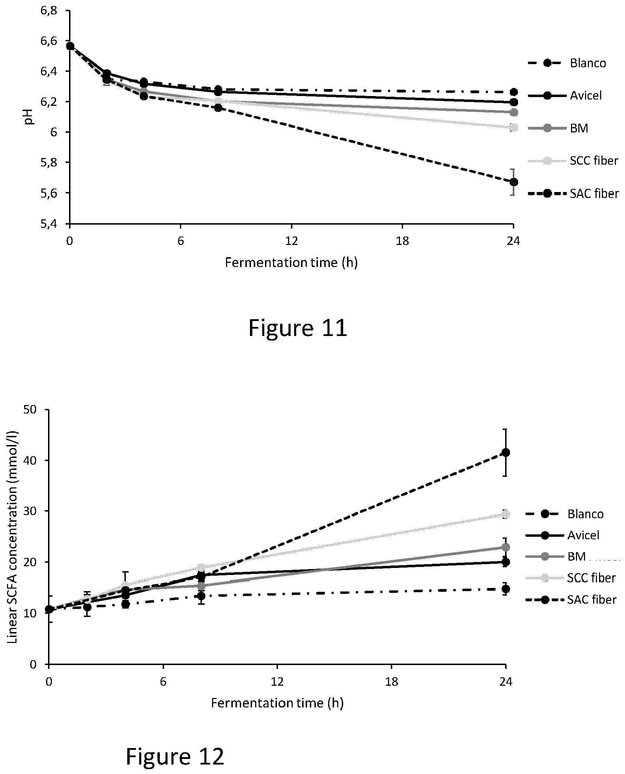

To translate this enhanced enzymatic accessibility into food functionality, the behavior of the celluloses in the human large intestine is evaluated with an *in vitro* approach. The pH during fermentation at 37°C of a fecal slurry with the addition of untreated, ball-milled, SCC or SAC fibers from Avicel is shown in Figure 11. SCFA production, induced by carbohydrate fermentation, will decrease the pH.

The fermentability of cellulose after 24 h is increased when the Avicel is ball milled once or ball-milled and subsequently hydrolyzed with citric acid. This shows that decreasing the crystallinity or decreasing the average DP of cellulose is improving the accessibility of cellulose for the gut microbiota. The largest pH drop is obtained when the SAC fibers were added. Also in this *in vitro* trial, decreasing both the DP and the crystallinity significantly decreases the pH of the lumen. This pH decrease is positive for human health since pathogen growth is repressed and proteolytic fermentation, which produces several toxic metabolites, is inhibited [den Besten et al. (2013) J. lipid research 54, 2325-2340].

The production of SCFA during this *in vitro* fermentation experiment is determined with gas chromatography. Acetate, butyrate and propionate are the main SCFA from carbohydrate fermentation in the colon and are named 'linear SCFA'. Figure 12 shows that more linear SCFA were produced in the fecal slurry upon the addition of SAC fibers. The final concentration of acetate, propionate and butyrate in the fecal slurry with SAC fibers was 41,54 mmol/l, while the control fecal slurry had only 14,85 mmol/l linear SCFA. The minimal degree of fermentability (MDOF) of the SAC fibers can be calculated based on the difference between the linear SCFA mass in the control fecal slurry and in the fecal slurry with SAC addition. At least 45,8% of the mass of the SAC fibers was fermented into acetate, propionate and butyrate after 24h, while the MDOF of untreated Avicel and BM Avicel was only 7,6% and 13,3%, respectively. The actual fermentation degree will always be even higher than the MDOF since part of the mass will also be converted to lactate, water, $H_2$,.. which are not analyzed in this experiment [Glenn et al. (1995) J. Nutrition 125(6), 1401-1412]. SCC fibers were also able to increase the SCFA production (MDOF = 30,8%), but the further mechanical treatment after the hydrolysis results in the highest fermentation degree.

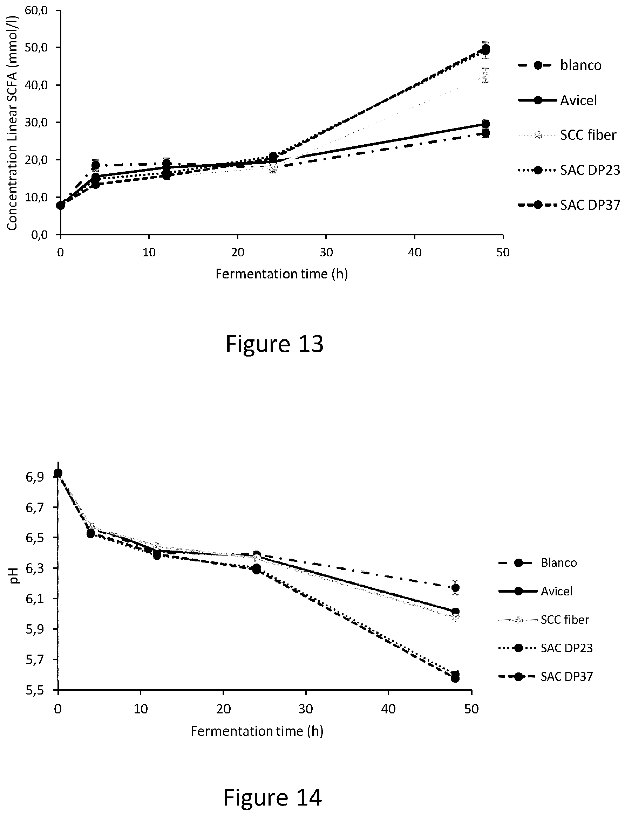

In a second *in vitro* fermentation trial, the influence of DP within the low DP ranges was investigated, comparing a SAC fiber with an average DP of 23 AGU (SAC DP23) with a SAC fiber with an average DP of 37 (SAC DP37) (figure 13). Contrary to previous experiments, the ball mill post-treatment was performed for 1h instead of 30 minutes. The pH plot illustrates that the cellulose fibers are mainly fermented between 24 and 48h, while in the previous experiment, the largest pH drop was obtained between 8 and 24h. This difference is likely due to the use of the feces of different donors. However, also here, SAC fibers are better fermentable than the untreated Avicel or SCC fiber.

Also in the production of linear SCFA, the same effect can be detected: The SCFA production follows the same kinetics as the pH progress, and the DP difference between 23AGU and 37AGU is not sufficient to make significant differences in SCFA production during an in vitro fermentation trial. The addition of untreated Avicel did not significantly increase the SCFA content in the fecal slurry, which means that the fermentation of the Avicel is negligible again (MDOF of 5.5%). However, the addition of the SAC fibers could increase the content of linear SCFA in the fecal slurry, resulting in an MDOF 46% for both SAC fibers. Despite the individual variability of the feces donors, the degree of fermentation is comparable with the previous *in vitro* fermentation experiment.

Next to the absolute production of acetate, propionate and butyrate, the relative production of this linear SCFA compared to the branched SCFA is investigated. These branched SCFA (isovalerate and isobutyrate) are products of protein fermentation, which are to be avoided as they may be further metabolized in the human body into toxic metabolites (*e.g.* phenolic compounds, ammonia, amines,...) [Hughes et al., (2000) Current issues in intestinal microbiology 1,51-58**]**. In Table 4, the ratio of branched SCFA from protein fermentation (isovalerate and isobutyrate) to total SCFA is shown for the fecal slurry after 48h of fermentation. The addition of SAC fibers decreases this proportion from 8.45% to 6.01% or 6.04%, demonstrating that these fibers inhibit protein fermentation, which has a positive effect on colon health. The incorporation of the SCC fiber also resulted in a decreased relative protein fermentation as well, but the greatest effect is obtained if Short amorphous cellulose is incorporated.

**Table 5: ratio of isovalerate and isobutyrate versus total SCFA**

| Cellulose sample | Branched SCFA/Total SCFA |
|---|---|
| **blanco** | 8,45 |
| **Avicel** | 7,88 |
| **SCC Fiber** | 6,55 |
| **SAC DP 23** | 6,01 |
| **SAC DP 37** | 6,04 |

The different linear SCFA all have a distinct metabolic function in the human body. The physiologic effects of the SCFA furthermore depend on the relative amounts of acetate, propionate and butyrate [Wong et al. (2006) J. Clinical Gastroenterology 40(3), 235-243]. The production of these SCFA is shown in Figure 14. Wong *et al.* stated that the average proportion acetate: propionate: butyrate is 3:1:1 on average, which is also clear from the figure. However, the butyrate production is for these fecal slurries always lower than the propionate production. In the first phase of the experiment (0-24h), mainly the acetate production is high, while the butyrate and propionate production is rather limited. Between 24 and 48h, acetate is produced, together with higher amounts of butyrate and propionate. Propionate and butyrate are produced from secondary fermentation and consequently depend on the acetate content in the fecal slurry, which explains this behavior [Wong *et al.,* cited above]. When the ratio butyrate/linear SCFA is calculated, it is clear that the addition of SAC fibers also had an impact on the relative amounts of butyrate compared to the acetate production. Moreover, when adding SAC DP23 or SAC DP37, the ratio butyrate/linear SCFA is equal to 17.6% and 15.7%, respectively, while for the control fecal slurry, this ratio is only 13.1%. Enhanced production of butyrate is correlated to increased colon health and lower risk on inflammation, as the butyrate is the main energy source for the gut cells. Next to the decrease in (lumen) pH, inhabitation of the protein fermentation and enhanced production of SCFA, adding the SAC fibers to a fecal slurry also causes an increase in the relative amount of butyrate.

**Claims**

1. A method of producing a microcrystalline cellulose, the method comprising the steps of:

    a) providing a composition comprising at least 60 % (w/w) cellulose, wherein the cellulose has an average Degree of Polymerization (DP) of between 20 and 100,
    b) mechanically treating said composition until the crystallinity index of said cellulose is below 0.5 and the degree of polymerization is below 100.

2. The method according to claim 1, wherein in step a, said composition comprises at least 70, at least 80, or at least 90 % (w/w) cellulose.

3. The method according to claim 1 or 2, wherein in step b) the composition is treated until the crystallinity index of said cellulose is below 0.45, below 0.40 below 0.35 or below 0.33.

4. The method according to any one of claims 1 to 3, wherein in step b) the composition is treated until the average degree of polymerization is below 80, below 60, below 50 below 40 or below 30.

5. The method according to any one of claims 1 to 4, wherein in step b) the composition is treated until the crystallinity index of said cellulose is below 0.33 and the average degree of polymerization is below 50.

6. The method according to any one of claims 1 to 5, wherein the composition in step a) is obtained via a method of mechanically treating a composition comprising cellulose and a subsequent method of hydrolyzing said mechanically treated composition.

7. A composition comprising at least 60 wt % cellulose, wherein the cellulose has a degree of Polymerization (DP) below 100 and a crystallinity index below 0,5.

8. The composition according to claim 7, wherein the crystallinity index of said cellulose is below 0.45, below 0.40 below 0.35 or below 0.33.

9. The composition according to claim 7 or 8, wherein the average degree of polymerization of said cellulose is below 80, below 60, below 50, below 40 or below 30.

10. The composition according to any one of claims 7 to 9, wherein the crystallinity index of said cellulose is below 0.33 and the average degree of polymerization is below 50.

11. The composition according to any one of claimd 7 to 10 comprising at least 70, at least 80 or at least 90 % cellulose.

12. A food product comprising between 1 and 50 wt % of a composition according to any one of claims 7 to 11.

13. The food product according to claim 12, which is a cereal product.

14. Use of a composition according to any one of claims 7 to 11 as a dietary fiber in a food composition.

Figure 1

Figure 2

Figure 3

Figure 4

Figure 6

Figure 5

Figure 7

Figure 8

Figure 9

Figure 10

Figure 11

Figure 12

Figure 13

Figure 14

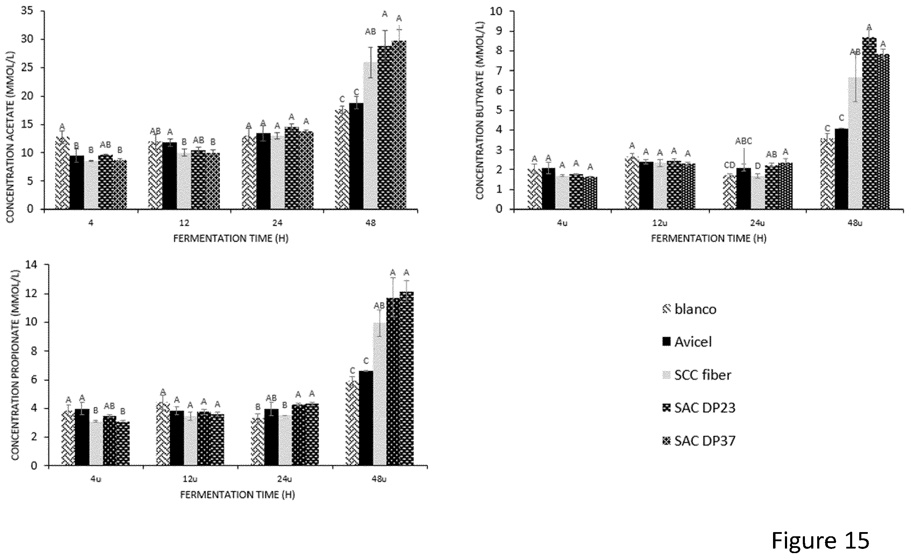

Figure 15

Europäisches
Patentamt

European
Patent Office

Office européen
des brevets

# EUROPEAN SEARCH REPORT

Application Number

EP 21 20 2228

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 357 467 A (SACHETTO JEAN-PIERRE ET AL) 2 November 1982 (1982-11-02) * claims; examples; tables * | 1-14 | INV. C08B15/02 C08L1/04 |
| A | PALA ET AL: "Enzymatic depolymerisation of cellulose", CARBOHYDRATE POLYMERS, APPLIED SCIENCE PUBLISHERS , LTD BARKING, GB, vol. 68, no. 1, 7 February 2007 (2007-02-07), pages 101-108, XP005878267, ISSN: 0144-8617, DOI: 10.1016/J.CARBPOL.2006.07.015 * paragraph [03.1]; table 1 * | 1-14 | |
| A | MATTONAI MARCO ET AL: "Effect of ball-milling on crystallinity index, degree of polymerization and thermal stability of cellulose", BIORESOURCE TECHNOLOGY, ELSEVIER, AMSTERDAM, NL, vol. 270, 7 September 2018 (2018-09-07), pages 270-277, XP085511085, ISSN: 0960-8524, DOI: 10.1016/J.BIORTECH.2018.09.029 * figures; table 1 * | 1-14 | |

TECHNICAL FIELDS
SEARCHED        (IPC)

C08B
C08L

The present search report has been drawn up for all claims

| Place of search | Date of completion of the search | Examiner |
|---|---|---|
| Munich | 23 March 2022 | Vaccaro, Eleonora |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 166 576 A1**

## ANNEX TO THE EUROPEAN SEARCH REPORT
## ON EUROPEAN PATENT APPLICATION NO.

EP 21 20 2228

This annex lists the patent family members relating to the patent documents cited in the above-mentioned European search report.
The members are as contained in the European Patent Office EDP file on
The European Patent Office is in no way liable for these particulars which are merely given for the purpose of information.

23-03-2022

| Patent document cited in search report | | Publication date | Patent family member(s) | | Publication date |
|---|---|---|---|---|---|
| US 4357467 | A | 02-11-1982 | EP | 0020469 A1 | 07-01-1981 |
| | | | IT | 1125574 B | 14-05-1986 |
| | | | US | 4357467 A | 02-11-1982 |
| | | | WO | 8000843 A1 | 01-05-1980 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHEN.** *Biotechnology of Lignocellulose,* 2005
- **CHERUBINI.** *Energy Conversion and Management,* 2010, vol. 51 (7), 1412-1421
- **BOZELL ; PETERSEN.** *Green Chemistry,* 2010, vol. 12 (4), 5392
- **BRINCHI et al.** *Carbohydrate polymers,* 2013, vol. 94 (1), 154-169
- **AN et al.** *Cellulose,* 2016, vol. 23 (4), 2409-2420
- **XU et al.** *Biotechnology for Biofuels,* 2012, vol. 5 (1), 58
- **CUMMINGS et al.** *Gut,* 1984, vol. 25 (8), 805-810
- **DEN BESTEN et al.** *J. lipid res.,* 2013, vol. 54 (9), 2325-2340
- **WONG et al.** *J. Clin. Gastroenterology,* 2006, vol. 40 (3), 235-243
- Scientific Opinion on Dietary Reference Values for Carbohydrates and Dietary Fibre. *EFSA Journal,* 2010, vol. 8 (3), 1462
- **HEMDANE et al.** *Food Chemistry,* 2015, vol. 187, 280-289
- **SWENNEN et al.** *J. Sci. Food and Agriculture,* 2006, vol. 86 (11), 1722-1731
- **MIRHOSSEINI et al.** *Carbohydrate Polymers,* 2008, vol. 73 (1), 83-91
- **SEGA et al.** *Textile Res. J.,* 1959, vol. 29 (10), 786-94
- **GUSAKOV.** *Biotechnology and Bioengineering,* 2007, vol. 97 (5), 1028-1103
- **CHEN et al.** *Applied Energy,* 2015, vol. 150, 224-232
- **DE PRETER et al.** *Molecular nutrition & food research,* 2010, vol. 54 (12), 1791-1801
- **VAN DE WIELE et al.** *J. Applied Microbiology,* 2007, vol. 102 (2), 452-460
- **ZHAO et al.** *Biofuels, Bioproducts and Biorefining,* 2012, vol. 6 (4), 465-482
- **LIN et al.** *Applied biochemistry and biotechnology,* 2010, vol. 162 (7), 1872-1880
- **SHROTRI et al.** *Green Chemistry,* 2013, vol. 15 (10), 2761-2768
- **CALVINI.** *Cellulose,* 2005, vol. 12, 445-472
- **DENEYER et al.** *Green Chemistry,* 2016, vol. 18 (20), 5594-5606
- **LIAO et al.** *Molecular Catalysis,* 2020, vol. 487, 110883
- **DEN BESTEN et al.** *J. lipid research,* 2013, vol. 54, 2325-2340
- **GLENN et al.** *J. Nutrition,* 1995, vol. 125 (6), 1401-1412
- **HUGHES et al.** *Current issues in intestinal microbiology,* 2000, vol. 1, 51-58
- **WONG et al.** *J. Clinical Gastroenterology,* 2006, vol. 40 (3), 235-243